# EUROPEAN PATENT APPLICATION

(11) **EP 4 321 167 A1**
(43) Date of publication of application: **14.02.2024**
(21) Application number: 21936165.6
(22) Date of filing: 10.12.2021
(51) Int. Cl.: A61K 36/739, A61P 31/14

(54) **SANGUISORBA OFFICINALIS LINNE EXTRACT COMPOSITION INHIBITING 3CL PROTEASE AND RDRP ACTIVITY OF SARS-COV-2**

(30) Priority: 08.04.2021 KR 20210045995; 02.12.2021 KR 20210170899
(71) Applicant: QGenetics Co., Ltd., Seoul 02447 (KR)
(72) Inventor: CHANG, Mun Seog, Seoul 04427 (KR); LEE, Tae Hee, Daejeon 34965 (KR); LEE, Min Ji, Gwangmyeong-si Gyeonggi-do 14226 (KR); LEE, Seung Jae, Seongnam-si Gyeonggi-do 13206 (KR); KIM, Ha Young, Seoul 03638 (KR); MIN, Jung Bin, Seoul 05649 (KR); PARK, Ji Hye, Seoul 02454 (KR); CHOI, Won Hyung, Goyang-si Gyeonggi-do 10558 (KR); PARK, Wook Ha, Namyangju-si Gyeonggi-do 12248 (KR)
(74) Representative: Viering, Jentschura & Partner mbB Patent- und Rechtsanwälte
(86) International application number: PCT/KR2021/018706
(87) International publication number: WO 2022/215827

(57) **Abstract**

The present invention relates to a use of a novel Sanguisorba officinalis Linne extract to inhibit SARS-CoV-2 virus 3CL protease and RdRp activity. The composition including the Sanguisorba officinalis Linne extract of the present invention as an active ingredient inhibits 3CL protease and RdRp activity and thus is effective in preventing or treating infection by SARS-CoV-2 and a variant virus thereof.

## Description

### TECHNICAL FIELD

The present invention relates to the function and use of an extract of Sanguisorba officinalis Linne that inhibits the activity of 3C-like protease (3CL protease) and RNA-dependent RNA Polymerase (RdRp) possessed by SARS-CoV-2 virus.

### BACKGROUND ART

Sanguisorba officinalis Linne refers to burnet and is a perennial plant belonging to the Rosaceae family. It is distributed in Korea, China, Japan, Russia, etc., and is a wild herb that grows in mountains and fields. Since ancient times, Sanguisorba officinalis Linne has been used as a detoxifier and a reliever to burns, diarrhea, and respiratory and gastrointestinal diseases, and various effects of Sanguisorba officinalis Linne such as anti-allergy, anti-cancer, and antioxidant properties have been reported.

Generally, methanol, ethanol, or water is used as a solvent for extracting plants, and useful effects of a hot water extract of Sanguisorba officinalis Linne have been reported. Previous studies have reported an anticancer effect that inhibits cell proliferation of oral cancer, and an effect of enhancing immunopotentiation. Compared to other methods, the hot water extraction method allows for easily extracting natural substances and obtaining a large amount of useful ingredients. In addition, since it is an extraction method that does not use chemical components such as ethanol, it has low toxicity and the extracts have high efficacy and are safe for food intake. In addition, Sanguisorba officinalis Linne is a medicinal herb that grows in mountainous areas and has the advantage of being easy to cultivate, highly self-sustaining, and easily available.

Korean Patent Registration No. 10-17842540000 discloses 'a composition for preventing or treating sepsis comprising Sanguisorba officinalis extract as effective component' and Korean Patent Publication No. 2011-0117540 discloses 'a pharmaceutical composition for prevention or treatment of diseases related to helicobacter infection comprising extracts of sanguisorba officinalis as an effective component and a health food.' However, it has never been reported in the literature that an extract of Sanguisorba officinalis Linne is effective in preventing, ameliorating or treating SARS-CoV-2 (severe acute respiratory syndrome coronavirus 2), which is a novel virus.

The inventors completed the present invention by confirming that a Sanguisorba officinalis Linne extract has an effect of preventing or treating infection by SARS-CoV-2.

### DETAILED DESCRIPTION OF THE INVENTION

### TECHNICAL PROBLEM

Sanguisorba officinalis Linne refers to burnet and is a perennial plant belonging to the Rosaceae family. It is distributed in Korea, China, Japan, Russia, etc., and is a wild herb that grows in mountains and fields. Since ancient times, Sanguisorba officinalis Linne has been used as a detoxifier and a reliever to burns, diarrhea, and respiratory and gastrointestinal diseases, and various effects of Sanguisorba officinalis Linne such as anti-allergy, anti-cancer, and antioxidant properties have been reported.

Generally, methanol, ethanol, or water is used as a solvent for extracting plants, and useful effects of a hot water extract of Sanguisorba officinalis Linne have been reported. Previous studies have reported an anticancer effect that inhibits cell proliferation of oral cancer, and an effect of enhancing immunopotentiation. Compared to other methods, the hot water extraction method allows for easily extracting natural substances and obtaining a large amount of useful ingredients. In addition, since it is an extraction method that does not use chemical components such as ethanol, it has low toxicity and the extracts have high efficacy and are safe for food intake. In addition, Sanguisorba officinalis Linne is a medicinal herb that grows in mountainous areas and has the advantage of being easy to cultivate, highly self-sustaining, and easily available.

Korean Patent Registration No. 10-17842540000 discloses 'a composition for preventing or treating sepsis comprising Sanguisorba officinalis extract as effective component' and Korean Patent Publication No. 2011-0117540 discloses 'a pharmaceutical composition for prevention or treatment of diseases related to helicobacter infection comprising extracts of sanguisorba officinalis as an effective component and a health food.' However, it has never been reported in the literature that an extract of Sanguisorba officinalis Linne is effective in preventing, ameliorating or treating SARS-CoV-2 (severe acute respiratory syndrome coronavirus 2), which is a novel virus.

The inventors completed the present invention by confirming that a Sanguisorba officinalis Linne extract has an effect of preventing or treating infection by SARS-CoV-2.

### TECHNICAL SOLUTION

To achieve the object described above, the present invention provides a composition for treating and/or preventing COVID-19 caused by SARS-CoV-2 through inhibition of 3CL protease and RdRp activity, comprising a Sanguisorba officinalis Linne extract as an active ingredient.

In addition, provided is a health functional food composition for preventing and/or ameliorating infection by SARS-CoV-2, comprising a Sanguisorba officinalis Linne extract as an active ingredient.

In addition, provided is a method for preparing a composition for preventing or treating infection by SARS-CoV-2, the method comprising: drying Sanguisorba officinalis Linne;
extracting the dried Sanguisorba officinalis Linne with a solvent at 30 to 120°C; and
freeze-drying the extracted solution to produce a powder.

### ADVANTAGEOUS EFFECTS

The composition of the present invention, comprising a Sanguisorba officinalis Linne extract as an active ingredient, has a therapeutic effect on COVID-19 caused by SARS-CoV-2 by inhibiting SARS-CoV-2-specific 3CL protease and RdRp activity.

### DESCRIPTION OF THE DRAWINGS

FIG. 1 is a diagram showing the results of inhibiting the activity of 3CL protease in a Sanguisorba officinalis Linne extract group, a negative control group, and a positive control group in an *in vitro* assay. **, p < 0.01; ***, p < 0.001.
FIG. 2 is a diagram showing the results of inhibiting the activity of SARS-CoV-2 3CL protease in a Sanguisorba officinalis Linne extract group, a negative control group, and a positive control group in a cell-based assay. *, p < 0.05; **, p < 0.01; ***, p < 0.001.
FIG. 3 is a diagram showing the results of inhibiting the activity of SARS-CoV-2 RdRp in a Sanguisorba officinalis Linne extract group, a negative control group, and a positive control group in an *in vitro* assay. **, p < 0.01; ***, p < 0.001.
FIG. 4 is a diagram showing the results of inhibiting the activity of SARS-CoV-2 proliferation in a Sanguisorba officinalis Linne extract group, a negative control group, a positive control group 1 (GC376), and a positive control group 2 (Remdesivir) at a cellular level.
FIG. 5 is a diagram showing the results of inhibiting weight loss in a Sanguisorba officinalis Linne extract group, a negative control group, a positive control group in SARS-CoV-2 infected hamsters. *, p < 0.05; **, p < 0.01; ***, p < 0.001.
FIG. 6 is a diagram confirming the results of inhibiting virus proliferation in a Sanguisorba officinalis Linne extract group, a negative control group, a positive control group in lung tissues of SARS-CoV-2 infected hamsters through RT-PCR. *, p < 0.05
FIG. 7 is a diagram confirming the results of inhibiting virus proliferation in a Sanguisorba officinalis Linne extract group, a negative control group, a positive control group in lung tissues of SARS-CoV-2 infected hamsters through a plaque assay. M, mean value.
FIG. 8 is a diagram confirming the results of inhibiting virus proliferation in a Sanguisorba officinalis Linne extract group, a negative control group, a positive control group in lung tissues of SARS-CoV-2 infected hamsters through tissue staining.
FIG. 9 is a diagram showing the results of inhibiting inflammation inhibition in a Sanguisorba officinalis Linne extract group, a negative control group, a positive control group in lung tissues of SARS-CoV-2 infected hamsters. *, p < 0.05.

### BEST MODE

The present invention relates to a use of a Sanguisorba officinalis Linne extract to inhibit SARS-CoV-2 virus 3CL protease and RdRp activity.

### MODE FOR INVENTION

Hereinafter, the present invention will be described in detail.

The present invention provides a pharmaceutical composition for preventing or treating infection by SARS-CoV-2, comprising a Sanguisorba officinalis Linne extract as an active ingredient.

SARS-CoV-2 is a virus that causes a respiratory syndrome by infection and is designated as a class 1 notifiable infectious disease. It is currently causing a global pandemic and is causing very serious illness in the elderly people at the age of 80s or more with a mortality rate of up to 25%. Intranasal invasion of SARS-CoV-2 through routes such as airborne droplets or in-person contact is considered the main cause of transmission.

SARS-CoV-2 that is introduced to the human body binds to the ACE2 (Angiotensin-Converting Enzyme 2) receptor on the cell surface, infiltrates into the cell, and divides and proliferates to generates a large amount of new viruses (Jackson CB, et al. Mechanisms of SARS-CoV-2 entry into cells. Nat Rev Mol Cell Biol. 2021 5;1-18). The generated SARS-CoV-2 is again secreted out of the cell and proliferates by repeating the same process of reinfiltrating into other cells. Therefore, the mechanisms involved in cellular infection, intracellular replication, and proliferation of SARS-CoV-2 are major targets for the therapeutic development.

SARS-CoV-2 is generating many variants, including the delta type, and these variant viruses are mainly caused by genetic mutations in the spike domain, which plays the role of infiltrating host cells (Harvey, W.T., et al. SARS-CoV-2 variants, spike mutations and immune escape. Nat Rev Microbiol 19, 2021 409-424) .

SARS-CoV-2 (severe acute respiratory syndrome coronavirus 2) is an acute severe respiratory disease coronavirus 2, which was first identified in 2019 and is classified as a positive-sense single-stranded RNA virus. The disease caused by this virus was named as coronavirus disease 2019, which is abbreviated as COVID-19.

The disease caused by SARS-CoV-2 may be coronavirus disease-19. The coronavirus disease-19 may be a respiratory disease. The respiratory disease may be pneumonia. The symptoms of COVID-19 may be at least one of fever, malaise, cough, dyspnea, phlegm, sore throat, headache, hemoptysis, nausea, and diarrhea.

The SARS-CoV-2 virus may include a variant thereof.

The variant may be characterized by a mutation that has occurred in the spike protein of the SARS-CoV-2 virus.

Meanwhile, other than mutations in the spike protein of the SARS-CoV-2 virus, no viruses with mutated genes in the regions involved in the activity of 3CL protease and RdRp have been reported, and so it is known that an inhibitor targeting the activity of 3CL protease and RdRp may be expected to have the same therapeutic efficacy to variant viruses.

The composition comprising a Sanguisorba officinalis Linne extract as an active ingredient may be characterized in that it decreases or inhibits 3CL protease (3C-like protease) activity.

The composition comprising a Sanguisorba officinalis Linne extract as an active ingredient may be characterized in that it SARS-CoV-2-specifically inhibits 3CL protease and RdRp activity.

The 3CL protease, along with papain-like protease, is encoded in the SARS-CoV-2 gene and is an essential protein for producing functional proteins that are necessary for the proliferation of SARS-CoV-2 in the body (Anirudhan V, et al. Targeting SARS-CoV-2 viral proteases as a therapeutic strategy to treat COVID-19. J Med Virol. 2021 93(5): 2722-2734). The functional proteins required for the proliferation encoded by SARS-CoV-2 are expressed in the form of a single polypeptide by using the protein synthesis system of the host cell. What is unique is that each protein that is covalently linked within this polypeptide does not perform its original function but it can acquire a function when it exists independently by being cleaved by a protease (Anirudhan V, et al. Targeting SARS-CoV -2 viral proteases as a therapeutic strategy to treat COVID-19. J Med Virol. 2021 93(5): 2722-2734). It is known that 3CL protease is known to produce functional proteins by acting on 11 cleavage sites that are present in a polypeptide, while papain-like protease is known to produce functional proteins by acting on 3 cleavage sites (Anirudhan V, et al. Targeting SARS-CoV-2 viral proteases as a therapeutic strategy to treat COVID-19. J Med Virol. 2021 93(5): 2722-2734). Therefore, when 3CL protease is inhibited, the production of many functional proteins may be inhibited compared to papain-like protease, and so this enzyme may be a major target for the development of a SARS-CoV-2 treatment agent.

The composition may be characterized that it decreases or inhibits RdRp (RNA-dependent RNA polymerase) activity.

The RdRp is an enzyme that is responsible for the replication and transcription of the RNA genome in most RNA viruses, including SARS-CoV-2 (Aftab, S.O., et al. Analysis of SARS-CoV-2 RNA-dependent RNA polymerase as a potential therapeutic drug target using a computational approach. J Transl Med 2020 18, 275) .

The present inventors completed the present invention by screening substances that inhibit the enzymatic activities of SARS-CoV-2-specific 3CL protease and RdRp by using hot water extracts of 223 herbal medicines to develop SARS-CoV-2 treatment and prevention agents.

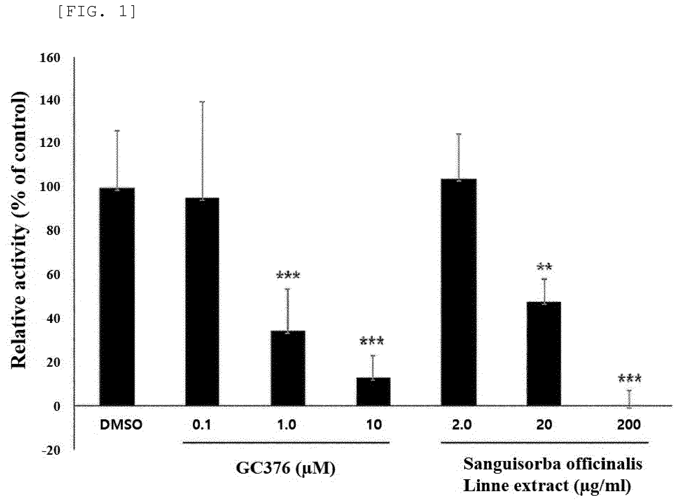

In a specific embodiment of the present invention, Sanguisorba officinalis Linne was used to prepare a Sanguisorba officinalis Linne extract, which was used in *in vitro* assays, cell tests, and animal tests using hamsters. As a result, compared to the negative control group in which the Sanguisorba officinalis Linne extract was not used, it showed an effect of decreasing the proliferation of SARS-CoV-2 through the inhibition of 3CL protease and RdRp (see FIGS. 1 to 9). Therefore, it was confirmed that the Sanguisorba officinalis Linne extract of the present invention may be usefully used as a pharmaceutical composition for preventing or treating infection caused by SARS-CoV-2 virus.

In addition, as can be seen in Example 2 below, the 3CLglow assay kit (Montana Molecular, USA) used for this experiment is based on a GFP fusion protein in which a domain cleaved by 3CL protease is attached to the c-terminal of the GFP protein. While a GFP fusion protein does not show fluorescence, a pure GFP protein of which c-terminal fusion domain has been cleaved by 3CL protease shows fluorescence. In other words, when 3CL protease is inhibited, fluorescence generation from the GFP fusion protein also decreases. Therefore, the inhibition of the activity of SARS-CoV-2 3CL protease by a Sanguisorba officinalis Linne extract was confirmed by the decrease of fluorescence generation.

In addition, as can be seen in Example 5 below, as a positive control, an MC vehicle containing molnupiravir, an RdRp inhibitor, in an amount of 100 mg/kg to 300 mg/kg, preferably 250 mg/kg, may be orally administered to hamsters, but not limited thereto. When the content of molnupiravir is less than the range described above, the inhibitory effect of SARS-CoV-2 in hamsters is minimal, and when it is more than the range described above, the benefit in terms of cost is small. The reason for using a high concentration (250 mg/kg) at this time is because, unlike ferrets for which low-concentration (15 mg/kg) molnupiravir is used (Cox RM et al. Therapeutically administered ribonucleoside analogue MK-4482/EIDD-2801 blocks SARS-CoV-2 transmission in ferrets. Nat Microbiol. 2021 6(1):11-18), high-concentration molnupiravir exhibits an effect of inhibiting SARS-CoV-2 in hamsters (Rosenke K et al. Orally delivered MK-4482 inhibits SARS-CoV-2 replication in the Syrian hamster model. Nat Commun. 2021 12(1):2295).

The Sanguisorba officinalis Linne extract may be extracted with a solvent that is water, alcohol, or a mixture thereof.

The solvent may be water, ethanol, a lower alcohol having 1 to 5 carbon atoms, or an aqueous solution of a lower alcohol having 1 to 5 carbon atoms, but is not limited thereto.

When extraction is performed by using the solvent above, the extraction temperature is 20°C to 130°C, preferably 30°C to 120°C, and more preferably 90°C to 110°C, but is not limited thereto.

The pharmaceutical composition may include a pharmaceutically acceptable carrier, excipient, or diluent. The term "pharmaceutically acceptable" in the present invention means that the composition exhibits non-toxic properties to cells or humans exposed to the composition. The composition containing a pharmaceutically acceptable carrier may be in various oral or parenteral formulations. When formulated, it can be prepared by using commonly used diluents or excipients such as fillers, extenders, binders, wetting agents, disintegrants, and surfactants. The carriers, excipients, and diluents may be at least one selected from the group consisting of lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, physiological saline, methyl hydroxy benzoate, propyl hydroxy benzoate, talc, magnesium stearate, mineral oil, dextrin, calcium carbonate, propylene glycol, and liquid paraffin, but are not limited thereto, and all common carriers, excipients, or diluents may be used. The ingredients may be added independently or in combination to the Sanguisorba officinalis Linne extract, which is an active ingredient.

The pharmaceutical composition may have any one formulation selected from the group consisting of tablets, pills, powder, granules, capsules, suspensions, oral liquids, emulsions, syrup, sterilized aqueous solutions, non-aqueous solutions, suspensions, emulsions, freeze-dried formulations, and suppositories.

As a base for the suppository, witepsol, macrogol, tween 60, cacao butter, lauren butter, glycerol gelatin or the like may be used.

The pharmaceutical composition may have any one formulation selected from the group consisting of powder, granules, tablets, capsules, and liquid form.

The pharmaceutical dosage form of the extract may be used alone or in combination with other pharmaceutically active compounds as well as in appropriate combinations.

The pharmaceutical composition may be administered orally and may be a solid formulation or a liquid formulation. Solid formulations for oral administration include tablets, pills, powder, granules, capsules or the like, and these solid formulations are prepared by mixing the extract with at least one excipient, such as starch, calcium carbonate, and sucrose or lactose, gelatin or the like. In addition to simple excipients, lubricants such as magnesium stearate and talc are also used. Liquid formulations for oral administration include suspensions, oral solutions, emulsions, and syrup, and in addition to commonly used simple diluents such as water and liquid paraffin, various excipients such as wetting agents, sweeteners, fragrances, and preservatives may be included. Formulations for parenteral administration include sterile aqueous solutions, non-aqueous solutions, suspensions, emulsions, freeze-dried formulations, and suppositories. Non-aqueous solvents and suspensions include propylene glycol, polyethylene glycol, vegetable oil such as olive oil, and injectable ester such as ethyl oleate.

The pharmaceutical composition of the present invention may be administered in a pharmaceutically effective amount. There are no particular restrictions on the administered dose, and it may vary depending on the body absorption, body weight, the patient's age, gender, health conditions, diet, administration time, administration method, excretion rate, and severity of disease. The pharmaceutical composition of the present invention is manufactured by considering the effective dose range, and the unit dosage form formulated in this manner may be administered by a specialized administration method according to the judgment of an expert who monitors or observes the administration of the drug as needed or according to an individual's needs or may be administered several times in a regular time interval. The administration may preferably be performed once a day, or may be performed several times.

The Sanguisorba officinalis Linne extract may be characterized in that it may be extracted from roots of Sanguisorba officinalis L., roots of Sanguisorba longifolia, or a mixture thereof.

In addition, the present invention provides a food composition for preventing or ameliorating infection by SARS-CoV-2 or diseases caused thereby, comprising a Sanguisorba officinalis Linne extract as an active ingredient.

The food may be characterized in that it is one or more of health functional food or beverage.

When using the Sanguisorba officinalis Linne extract as a food additive, the Sanguisorba officinalis Linne extract may be added as is or used together with other foods or food ingredients, and may be used appropriately according to conventional methods. The mixing amount of the active ingredient may be appropriately determined depending on the purpose of use (prevention, health, or therapeutic treatment). Generally, when food or beverage is manufactured, the extract of the present invention may be added in an amount of 15 parts by weight or less, preferably 10 parts by weight or less, based on the raw materials. However, in the case of long-term intake for the purpose of health and hygiene or health control, the amount may be below the range described above, and since there is no problem in terms of safety, the active ingredient may be used in an amount more than the range described above.

There are no particular restrictions on the types of the health functional food. Examples of foods to which the Sanguisorba officinalis Linne extract may be added include meat, sausages, bread, chocolate, candies, snacks, confectionery, pizza, ramen, other noodles, gum, dairy products including ice cream, various soups, beverages, tea, and drink preparations, alcoholic beverages, and vitamin complexes, etc., and may include all health functional foods in the conventional sense.

The health functional beverage composition of the present invention may contain various flavoring agents or natural carbohydrates as additional ingredients, like conventional beverages. The natural carbohydrates described above include monosaccharides such as glucose and fructose, disaccharides such as maltose and sucrose, polysaccharides such as dextrin and cyclodextrin, and sugar alcohols such as xylitol, sorbitol, and erythritol. As a sweetener, natural sweeteners such as thaumatin and stevia extract or synthetic sweeteners such as saccharin and aspartame may be used.

In addition to the health functional food above, the health functional food of the present invention includes various nutritional supplements, vitamins, electrolytes, flavors, colorants, pectic acid and salts thereof, alginic acid and salts thereof, organic acids, protective colloidal thickeners, pH adjusters, stabilizers, and preservatives, glycerin, alcohols, carbonating agents used in carbonated drinks, etc. In addition, it may contain pulp for the manufacturing of natural fruit juice, fruit juice drinks, and vegetable drinks. These ingredients may be used independently or in combination. The ratio of these additives is not very important, but is generally selected in a range of 0.01 to 2 parts by weight per 100 parts by weight of the composition of the present invention.

The Sanguisorba officinalis Linne extract is preferably prepared by a preparation method comprising the steps described below, but is not limited thereto:
1) drying Sanguisorba officinalis Linne;
2) extracting the dried Sanguisorba officinalis Linne with a solvent at 30 to 120°C; and
3) freeze-drying the extracted solution to produce powder.

The present invention provides a method for producing a pharmaceutical composition for preventing or treating infection by SARS-CoV-2, comprising the steps of: 1) drying Sanguisorba officinalis Linne;
2) extracting the dried Sanguisorba officinalis Linne with a solvent at 30 to 120°C; and
3) freeze-drying the extracted solution to produce a powder.

The present invention provides a method for producing a health functional food composition for preventing or ameliorating infection by SARS-CoV-2, comprising the steps of: 1) drying Sanguisorba officinalis Linne;
2) extracting the dried Sanguisorba officinalis Linne with a solvent at 30 to 120°C; and
3) freeze-drying the extracted solution to produce a powder.

In the method described above, as the Sanguisorba officinalis Linne of step 1), Sanguisorba officinalis Linne or Sanguisorba longifolia that is either cultivated or commercially available may be used without limitation, and any of leaves, roots, stems, and branches may be used, but not limited to these. However, according to a preferred embodiment of the present invention, using roots Sanguisorba officinalis Linne or roots of Sanguisorba longifolia is most preferable.

In the method described above, the extraction solvent in step 1) is preferably water, alcohol, spirit, or a mixture thereof and an organic solvent. As the alcohol, a lower alcohol having 1 to 5 carbon atoms may be preferably used, and ethanol or methanol may be preferably used as a lower alcohol. As an extraction method, hot water extraction, shaking extraction, Soxhlet extraction, or reflux extraction may be preferably used, but it is not limited to these. Extract may be preferably performed by adding the extraction solvent in an amount 5 to 30 times the total weight of dried Sanguisorba officinalis Linne, and more preferably by adding 20 times the total weight thereof. The extraction temperature is preferably 20°C to 130°C, more preferably 90°C to 110°C, but is not limited thereto. In addition, the extraction time is preferably 2 to 48 hours, more preferably 15 to 30 hours, and most preferably 24 hours, but is not limited thereto. In addition, the number of times of extraction is preferably 1 to 5 times, more preferably 3 to 4 times, and most preferably 3 times, but is not limited thereto.

The Sanguisorba officinalis Linne extract is preferably a 30 to 95% alcohol extract, more preferably a 40 to 90% alcohol extract, and most preferably a 50 to 80% alcohol extract. In the case of the alcohol extract of less than 50%, the extraction efficiency may be lowered, and an intended effect of preventing or treating infection by the SARS-CoV-2 virus may not be exhibited. An alcohol extract exceeding 80% is not good because the production cost of the Sanguisorba officinalis Linne extract is increased.

The composition prepared by the preparation method above, comprising a Sanguisorba officinalis Linne extract as an active ingredient, may be characterized in that it exhibits an effect of preventing or treating infection by SARS-CoV-2 by inhibiting 3CL protease and RdRp activity.

Hereinafter, the present invention will be described in detail through examples and experimental examples.

However, the examples and experimental examples described below are merely illustrative of the present invention, and the content of the present invention is not limited by the examples and experimental examples described below.

### Example 1. Preparation of Sanguisorba officinalis Linne extract

Distilled water 20 times the weight of the total weight of the dried Sanguisorba officinalis Linne was added to dried Sanguisorba officinalis Linne (roots of Sanguisorba officinalis Linne) to perform cold soaking for 1 hour and 30 minutes. Afterwards, the solution was extracted by refluxing at 100°C for 1 hour and 30 minutes using a large decocting device. The extracted solution was filtered under reduced pressure to remove impurities, and then freeze-dried at -20°C to prepare powder. The freeze-dried Sanguisorba officinalis Linne powder in an amount of 0.2 g was added to 10 mL of distilled water to prepare a Sanguisorba officinalis Linne extract-containing solution (20 mg/ml), which was used in *in vitro* and cell tests. For animal testing using hamsters, 2 g of the freeze-dried Sanguisorba officinalis Linne powder was mixed with an excipient of 0.1 g and added to 10 mL distilled water to prepare a Sanguisorba officinalis Linne extract-containing solution.

### Example 2. Inhibition of activity of SARS-CoV-2-specific 3CL protease by Sanguisorba officinalis Linne extract

### 2-1. Analysis of 3CL protease activity using in vitro experiments

An analysis of the 3CL protease *in vitro* activity was performed at an *in vitro* level as described below. This experiment was performed by using a SARS-CoV-2 3CL protease (3C-like protease) inhibitor assay kit (BPS bioscience, USA). The test sample was subjected to a reaction with a substrate that exhibits fluorescence when bound to an active site of 3CL protease, and the fluorescence was measured by using a known method to confirm the effect of the sample to inhibit 3CL protease. For a negative control group, H₂O was used in the same volume as the sample, and for positive control groups for inhibiting 3CL protease activity, GC376 was used at 0.1, 1, and 10 µM, respectively. The reaction volume was 50 µl, and the treated Sanguisorba officinalis Linne extract was 2, 20, and 200 µg/ml, respectively.

As a result, as shown in FIG. 1, the IC50 was 26.8 µg/mL, and the Sanguisorba officinalis Linne extract inhibited the activity of SARS-CoV-2 3CL protease to a level equal to or higher than that of the positive control groups.

### 2-2. Analysis of 3CL protease activity analysis using cell-based analysis

The effect of the Sanguisorba officinalis Linne extract to inhibit 3CL protease activity was verified at a cellular level (cell-based assay) by using 293 cells. For this experiment, the 3CLglow assay kit (Montana Molecular, USA) was used.

The 293 cells (1×10³) were seeded in a 24-well plate. On the next day (18 to 24 hours later), the 293 cells were infected with baculovirus (1×10³) (Montana Molecular, USA) containing 3CL protease and a substrate thereof, which is a GFP fusion protein. Afterwards, the cells were treated with the Sanguisorba officinalis Linne extract at concentrations of 1, 10, and 100 µg/ml, respectively. The negative control group was treated with H₂O, and 0.1, 1, and 10 µM of GC376 were used for the positive control groups, respectively. After 24 hours, the inhibitory effect of the Sanguisorba officinalis Linne extract was observed through a fluorescence microscope (magnification 100X, scale bar=100 µm).

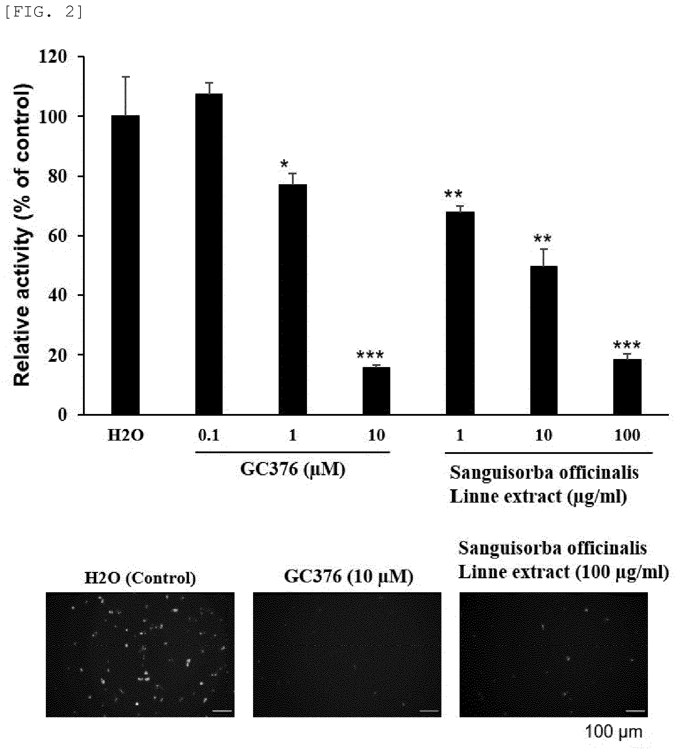

As a result, as shown in FIG. 2, the number of cells expressing fluorescence was significantly reduced in the Sanguisorba officinalis Linne extract-treated group, similar to the positive control group, GC376. The IC50 for SARS-CoV-2 3CL protease activity inhibition at the cellular level of the Sanguisorba officinalis Linne extract was 25.98 ug/ml, which was very similar to the results of the previous *in vitro* assay. Therefore, it was confirmed that the Sanguisorba officinalis Linne extract has a significant effect of inhibiting the activity of SARS-CoV-2 3CL protease at a cellular level.

### Example 3. Inhibition of SARS-CoV-2-specific RdRp activity by Sanguisorba officinalis Linne extract

RdRp (RNA-dependent RNA polymerase) *in vitro* activity analysis was performed at a test tube level as described below. The RdRp inhibitor assay kit (Profoldin, USA), the product used in the experiment, was purchased. The efficacy of the test samples was measured based on a substrate that exhibits fluorescence when bound to an RNA amplified by RdRp. For a negative control group for inhibition of RdRp activity, H₂O was used, and aurintricarboxylic acid (ATA) (Hung HC et al. Inhibition of enterovirus 71 replication and the viral 3D polymerase by aurintricarboxylic acid. J Antimicrob Chemother. 2010 65(4) : 676-83) was used for the positive control groups. The reaction volume was 50 µl, and the treated Sanguisorba officinalis Linne extract was 2, 20, and 200 µg/ml, respectively.

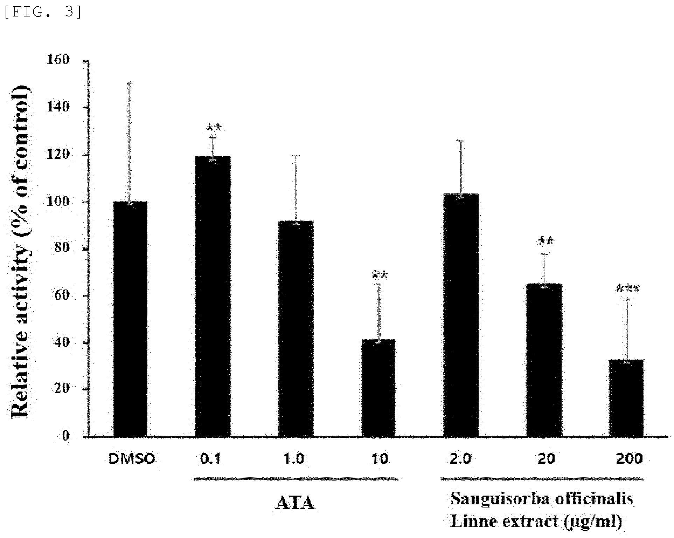

As a result, as shown in FIG. 3, the IC50 was 66.7 ug/ml, and the Sanguisorba officinalis Linne extract inhibited the activity of RdRp to a level equal to or higher than that of the positive control groups.

### Example 4. Inhibition of proliferation of SARS-CoV-2 by Sanguisorba officinalis Linne extract in VERO-E6 cells

VERO-E6 cells were dispensed into a 96-well plate at 8×10³ cells/100 µL per well and cultured overnight in a 5% CO₂ incubator at 37°C to reach approximately 80% confluency on the day of testing. VERO-E6 cells are cells derived from renal epithelial cells of African green monkeys and are used in virus infection experiments (Govorkova EA, et al. African green monkey kidney (Vero) cells provide an alternative host cell system for influenza A and B viruses. J Virol. 1996;70(8) :5519-5524). For the positive control groups of the present experiment, GC376, which is an inhibitor of 3CL protease, and remdesivir (Gilead Sciences, USA), which is an RdRp inhibitor, were used. In the case of the sample concentration of the Sanguisorba officinalis Linne extract, the non-toxic concentration to VERO-E6, 8.14 ug/ml, was set to be the highest concentration, and the sample was diluted double to reach the lowest concentration of 0.51 ug/ml. The diluted sample was mixed with SARS-CoV-2-containing culture medium (200 TCID₅₀), adjusted to a volume of 200 µl, and left in a CO₂ incubator at 37°C for 1 hour. Afterwards, the culture medium was removed from the cells and replaced with culture medium containing the sample and virus. After culturing for 3 days, the cytopathic effect (CPE), which induces cell necrosis by virus proliferation, was observed under a microscope, and the degree of CPE inhibition by the inhibition of virus proliferation was observed using the sample. This experiment was repeated three times, and the sample concentration for CPE inhibition was calculated as an average value.

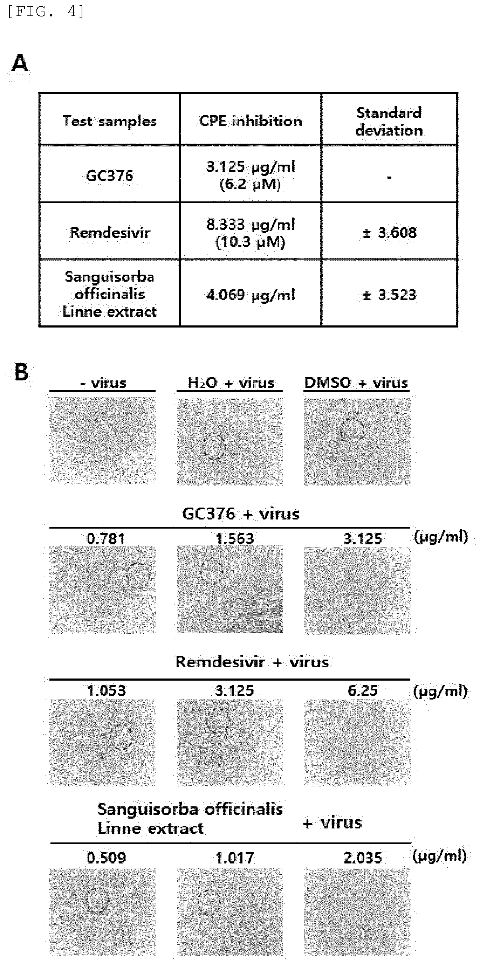

As a result, as shown in FIG. 4, the average CPE inhibition value of GC376 was 3.125 ug/ml, the average CPE inhibition value of remdesivir was 8.333 ug/ml, and the average CPE inhibition value of the Sanguisorba officinalis Linne extract was 4.069 ug/ml. Through this, it was confirmed that the Sanguisorba officinalis Linne extract of the present invention has a SARS-CoV-2 proliferation inhibitory effect that is twice as strong as remdesivir, which is a drug actually prescribed for SARS-CoV-2 infected patients, at a cellular level.

### Example 5. Inhibition of proliferation of SARS-CoV-2 by Sanguisorba officinalis Linne extract in hamster animal test

### 5-1. Construction of SARS-CoV-2 infection animal model using hamsters and oral administration of test substances

SARS-CoV-2 (NCCP43326), a known virus, was subcultured to prepare 10⁴ TCID50/mL virus in a PBS (phosphate buffered saline) solution. Afterwards, 100 µL of the SARS-CoV-2 virus solution was administered into the left nasal cavity of the hamster. Immediately after viral infection, an excipient, methylcellulose (MC) vehicle, was orally administered to a negative control group, and an MC vehicle containing high-concentration (250 mg/kg) molnupiravir (Merck, USA), which is an RdRp inhibitor, was administered to the positive control group. The Sanguisorba officinalis Linne extract was orally administered at 100 mg/kg by using an MC vehicle. Each test substance was administered twice daily. A Student's t-test was used to test the significance among the test substance administration groups, and the statistical analysis was performed by using Prism 7.04 (GraphPad Software Inc., San Diego, CA, USA). When the p value was less than 0.05, it was determined that there was statistical significance.

### 5-2. Inhibition of body weight loss by Sanguisorba officinalis Linne extract in SARS-CoV-2-infected hamsters

As in Example 5-1, SARS-CoV-2-infected hamsters were inoculated with the virus and the test substances were administered, and from that day, the body weight of each individual was measured by using an electronic scale every day for 7 days.

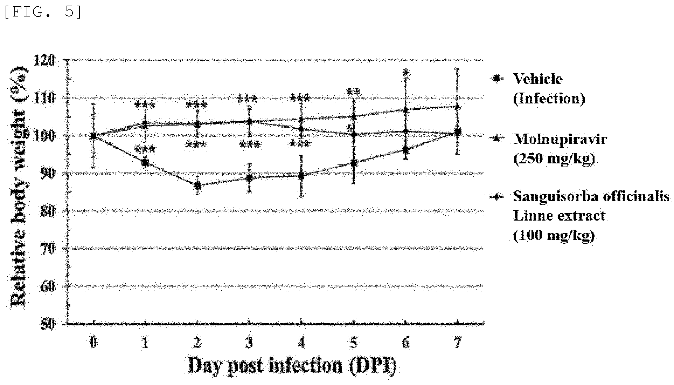

As a result, as shown in FIG. 5, when only the vehicle was administered to SARS-CoV-2 infected hamsters in the negative control group, the body weight decreased between 2 and 4 days of infection. On the contrary, in the group administered the Sanguisorba officinalis Linne extract, there was an effect of inhibiting the weight loss similar to the molnupiraviradministered group, which was a positive control group. Therefore, it was found that the Sanguisorba officinalis Linne extract strongly inhibited the division and proliferation of SARS-CoV-2 in the hamster animal test to restore homeostasis in the body, inducing normalization of the body weight.

### 5-3. Confirmation of inhibition of SARS-CoV-2 proliferation in hamster lungs by Sanguisorba officinalis Linne extract using RT-PCR technique

On the 3rd day post infection (3 DPI), a portion of the lesion was collected from the hamster's lung tissue, and a certain amount of Wizol^{™} (WizbioSolution, Seongnam, Korea) reagent was added, and then tissue grinding beads were used to grind the same. Then, the total RNA was extracted from the supernatant. The extracted total RNA was quantified by using Nanodrop (NanoDrop2000, Thermo scientific). From 1 µg of the total RNA, cDNA was synthesized by using the WizScript^{™} cDNA synthesis kit (WizbioSolution). After that, the synthesized cDNA was subjected to real-time RT-PCR using probeWizPure^{™} qPCR Master-UDG (WizbioSolution) using a SARS-CoV2 E gene-specific primer or an RdRp gene-specific primer. The primer sequences used for RT-PCT are listed in Table 1.

**[Table 1]**

| Item | Primer | SEQ ID NO. |
|---|---|---|
| E gene forward | 5'-ACAGGTACGTTAATAGTTAATAGCGT-3' | 1 |
| E gene reverse | 5'-ATATTGCAGCAGTACGCACACA-3' | 2 |
| RdRp gene forward | 5'-ATGAGCTTAGTCCTGTTG-3' | 3 |
| RdRp gene reverse | 5'-CTCCCTTTGTTGTGTTGT-3' | 4 |

Based on a standard curve created by using RNA derived from SARS-CoV-2 of a known copy number, the residual amount of virus derived from lung tissue was quantitatively analyzed as the number of copies of viral genes per ng or µg of the total RNA.

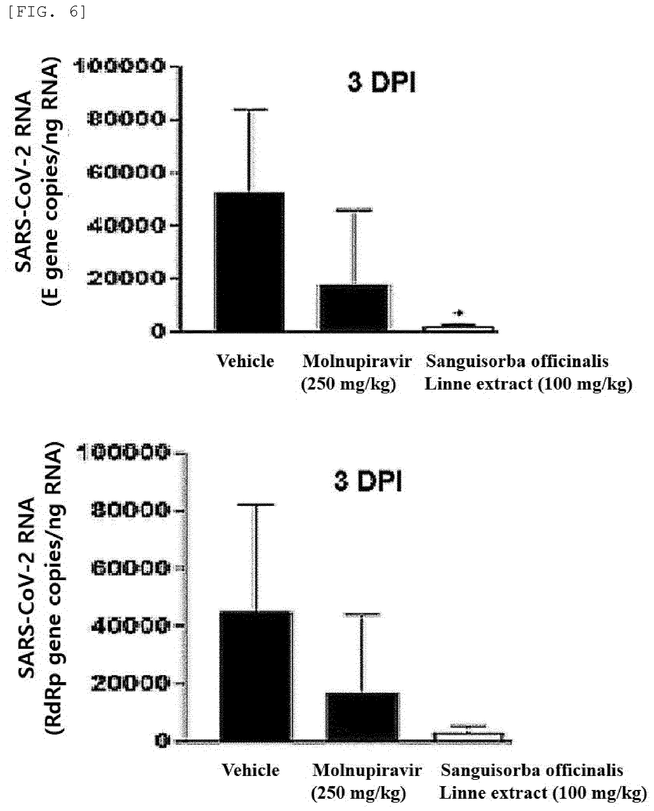

As a result, as shown in FIG. 6, it was confirmed that the Sanguisorba officinalis Linne extract has a significantly more efficacy of inhibiting the virus proliferation in the lung tissue of infected animals than molnupiravir, which decreases the proliferation of SARS-CoV-2 and thus inhibits the activity of RdRp.

### 5-4. Confirmation of inhibition of SARS-CoV-2 proliferation in hamster lungs by the Sanguisorba officinalis Linne extract by using Plaque assay

On the third day post infection (3 DPI), the lung tissue collected from hamsters) was homogenized by adding 10 µl of PBS per 1 mg. After centrifugation at 5,000 rpm for 3 minutes, the supernatant was recovered, adjusted to a volume of 200 µl by serial dilution with PBS, and then treated to Vero E6 cells growing in a 12-well plate. After shaking the virus every 15 minutes for 1 hour to evenly contact the Vero E6 cells, the supernatant was removed, and 1 ml of agarose overlay medium containing 1% agarose was added to each well. After confirming that the agarose overlay medium was hardened, the 12-well plate was turned over and cultured for 96 hours to observe the formation of plaques. The agarose overlay medium was removed and stained in crystal violet. The number of plaques per g of the tissue was calculated by visually comparing the number of plaques derived from viruses of a known copy number with the dilution factor of the sample.

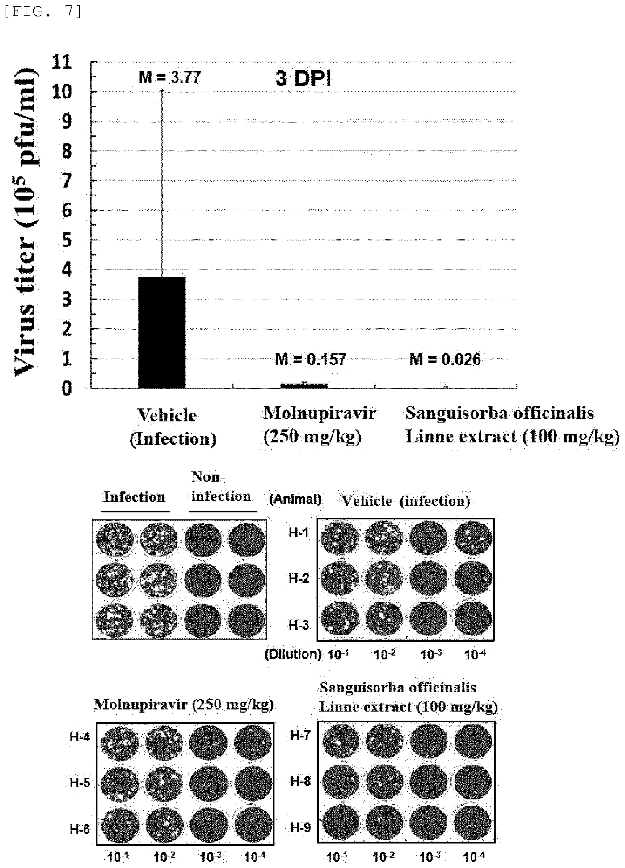

As a result, as shown in FIG. 7, it was found that the Sanguisorba officinalis Linne extract has a significantly more effective in inhibiting the proliferation of SARS-CoV-2 in hamster lung tissue than molnupiravir, which is an RdRp inhibitor.

### 5-5. Confirmation of inhibition of SARS-CoV-2 proliferation in hamster lungs by Sanguisorba officinalis Linne extract by using immunohistochemistry

On the third day post infection (3 DPI), the hamster lung tissue was collected, and paraffin blocks were prepared, and then the tissue was sectioned into a 5 um thickness. Afterwards, a known tissue staining method was carried out by using an antibody against a SARS-CoV-2-specific nucleocapsid (Cat# 40143-MM05; Sino Biological Inc.).

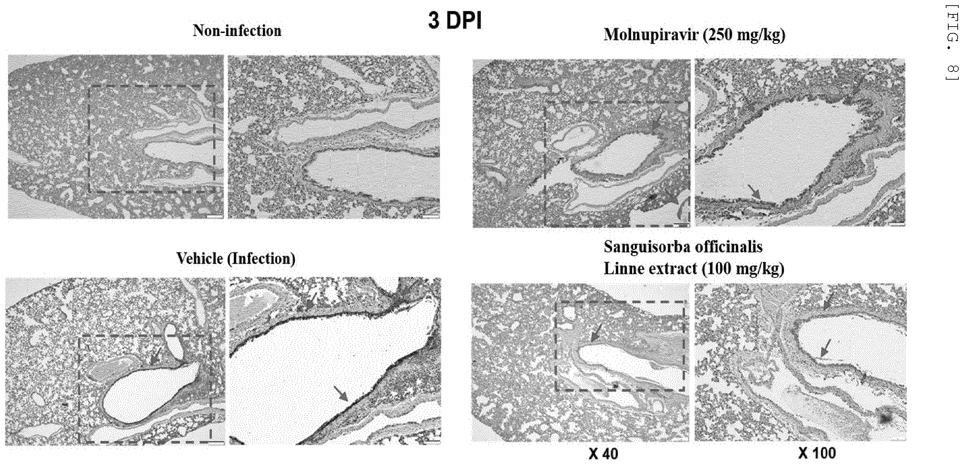

As a result, as shown in FIG. 8, in the vehicle-treated group, a strong brown reaction (nucleocapsid reaction) indicating infection by SARS-CoV-2 was observed in the epithelial cells within the lung tissue of the hamsters. On the contrary, in the case of the Sanguisorba officinalis Linne extract treatment group, the nucleocapsid reaction was similar to that of the positive control group of molnupiravir. Therefore, it was confirmed by using immunohistochemistry that the Sanguisorba officinalis Linne extract inhibits the division and proliferation of SARS-CoV-2 in hamster lung tissue.

### 5-6. Efficacy of Sanguisorba officinalis Linne extract to inhibit hamster lung inflammation caused by SARS-CoV-2 virus

On the third day post infection (3 DPI), three hamsters per group were autopsied, and the right lung tissue was fixed in a 10% NBF (neutralized buffered formalin) solution, and then histopathological examination was performed. A paraffin block was prepared by using the lung tissue fixed in 10% NBF, and the tissue was sectioned into a thickness of 5 um. Thereafter, inflammation scoring was evaluated by H&E (Hematoxylin & Eosin) staining and optical microscopic observation. The score was determined based on the literature by scoring from 0 to 3 points depending on the degree of inflammation (Reference 9). A value of 0 indicates absence of a lesion; one point was given to 10% or less; two points were given to 10% to 50%; and three points were given to 50% or more', and when bleeding or edema was observed, an additional 0.5 point was given.

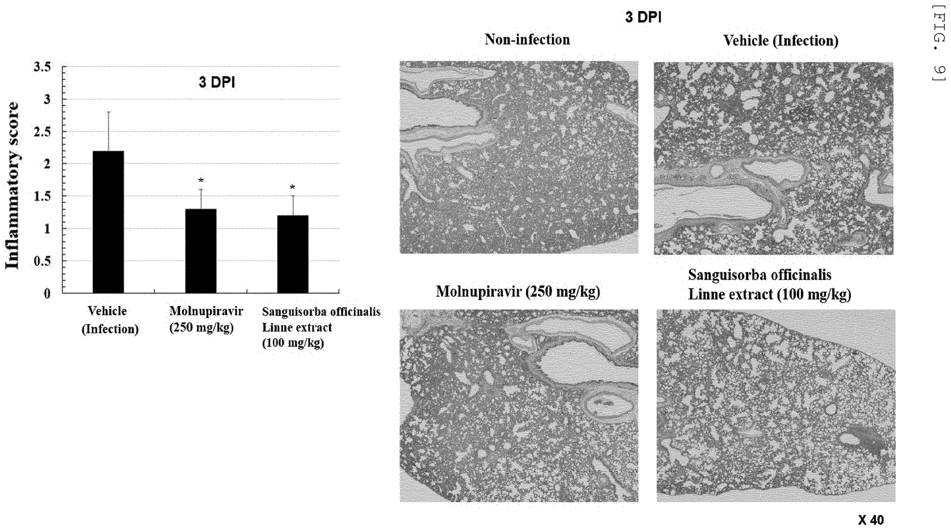

As a result, as shown in FIG. 9, it was confirmed that the Sanguisorba officinalis Linne extract has an effect equal to or greater than molnupiravir, a positive control group, in alleviating inflammation in hamster lung tissue caused by infection with SARS-CoV-2.

### INDUSTRIAL APPLICABILITY

Pharmaceutical products can be developed by using the use of the Sanguisorba officinalis Linne extract of the present invention to inhibit SARS-CoV-2 virus 3CL protease and RdRp activity.

## Claims

1. A pharmaceutical composition for preventing or treating infection by SARS-CoV-2 (severe acute respiratory syndrome coronavirus 2), comprising a Sanguisorba officinalis Linne extract as an active ingredient.

2. The pharmaceutical composition for preventing or treating infection by SARS-CoV-2 according to claim 1, **characterized in that** the SARS-CoV-2 comprises a variant thereof.

3. The pharmaceutical composition for preventing or treating infection by SARS-CoV-2 according to claim 2, **characterized in that** the variant has a mutation that has occurred in a spike protein of SARS-CoV-2 virus.

4. The pharmaceutical composition for preventing or treating infection by SARS-CoV-2 according to claim 1, **characterized in that** the composition decreases 3CL protease (3C-like protease) activity.

5. The pharmaceutical composition for preventing or treating infection by SARS-CoV-2 according to claim 1, **characterized in that** the composition decreases RdRp (RNA-dependent RNA polymerase) activity.

6. The pharmaceutical composition for preventing or treating infection by SARS-CoV-2 according to claim 1, **characterized in that** the Sanguisorba officinalis Linne extract is extracted with a solvent that is water, alcohol, or a mixture thereof.

7. The pharmaceutical composition for preventing or treating infection by SARS-CoV-2 according to claim 1, **characterized in that** the pharmaceutical composition comprises a pharmaceutically acceptable carrier, excipient, or diluent.

8. The pharmaceutical composition for preventing or treating infection by SARS-CoV-2 according to claim 1, **characterized in that** the pharmaceutical composition has any one formulation selected from the group consisting of tablets, pills, powder, granules, capsules, suspensions, oral liquids, emulsions, syrup, sterilized aqueous solutions, non-aqueous solutions, suspensions, emulsions, freeze-dried formulations, and suppositories.

9. The pharmaceutical composition for preventing or treating infection by SARS-CoV-2 according to claim 1, **characterized in that** the pharmaceutical composition has any one formulation selected from the group consisting of powder, granules, tablets, capsules, and liquid form.

10. The pharmaceutical composition for preventing or treating infection by SARS-CoV-2 according to claim 1, **characterized in that** the Sanguisorba officinalis Linne extract is extracted from roots of Sanguisorba officinalis L., roots of Sanguisorba longifolia, or a mixture thereof.

11. A health functional food composition for preventing or ameliorating infection by SARS-CoV-2, comprising a Sanguisorba officinalis Linne extract as an active ingredient.

12. The health functional food composition for preventing or ameliorating infection by SARS-CoV-2 according to claim 11, **characterized in that** the SARS-CoV-2 comprises a variant thereof.

13. The health functional food composition for preventing or ameliorating infection by SARS-CoV-2 according to claim 11, **characterized in that** the variant has a mutation that has occurred in a spike protein of SARS-CoV-2 virus.

14. The health functional food composition for preventing or ameliorating infection by SARS-CoV-2 according to claim 11, **characterized in that** the composition decreases 3CL protease (3C-like protease) activity.

15. The health functional food composition for preventing or ameliorating infection by SARS-CoV-2 according to claim 11, **characterized in that** the composition decreases RdRp (RNA-dependent RNA polymerase) activity.

16. A method for preparing a composition for preventing or treating infection by SARS-CoV-2, the method comprising:
drying Sanguisorba officinalis Linne;
extracting the dried Sanguisorba officinalis Linne with a solvent at 30 to 120°C; and
freeze-drying the extracted solution to produce a powder.

17. The method for preparing a composition for preventing or treating infection by SARS-CoV-2 according to claim 16, **characterized in that** the solvent is any one or more selected from the group consisting of water, alcohol, or a mixture thereof.

18. The method for preparing a composition for preventing or treating infection by SARS-CoV-2 according to claim 16, **characterized in that** the pharmaceutical composition comprises a pharmaceutically acceptable carrier, excipient, or diluent.

19. The method for preparing a composition for preventing or treating infection by SARS-CoV-2 according to claim 16, **characterized in that** the pharmaceutical composition has any one formulation selected from the group consisting of tablets, pills, powder, granules, capsules, suspensions, oral liquids, emulsions, syrup, sterilized aqueous solutions, non-aqueous solutions, suspensions, emulsions, freeze-dried formulations, and suppositories.

20. The method for preparing a composition for preventing or treating infection by SARS-CoV-2 according to claim 16, **characterized in that** the pharmaceutical composition has any one formulation selected from the group consisting of powder, granules, tablets, capsules, and liquid form.
